# EUROPEAN PATENT SPECIFICATION

(11) **EP 4 262 600 B1**
(45) Date of publication and mention of the grant of the patent: **19.02.2025**
(21) Application number: 21840962.1
(22) Date of filing: 21.12.2021
(51) Int. Cl.: A61B 18/12, A61B 18/00

(54) **ELECTRONIC APPARATUS FOR DELIVERING COHERENT SINE BURST IRREVERSIBLE ELECTROPORATION ENERGY TO A BIOLOGICAL TISSUE**
ELEKTRONISCHE VORRICHTUNG ZUR ABGABE VON IRREVERSIBLER ELEKTROPORATIONSENERGIE MIT KOHÄRENTEM SINUSBURST AN EIN BIOLOGISCHES GEWEBE
APPAREIL ÉLECTRONIQUE PERMETTANT DE DÉLIVRER UNE ÉNERGIE D'ÉLECTROPORATION IRRÉVERSIBLE À SALVE SINUSOÏDALE COHÉRENTE À UN TISSU BIOLOGIQUE

(30) Priority: 21.12.2020 WO PCT/US2020/066444
(43) Date of publication of application: 25.10.2023
(73) Proprietor: ARGA' MEDTECH SA, 1066 VD Epalinges (CH)
(72) Inventor: SHERMAN, Marshall, Cardiff by the Sea, California 92007 (US)
(74) Representative: Eisenführ Speiser
(86) International application number: PCT/EP2021/087130
(87) International publication number: WO 2022/136458

(56) References cited:
- WO-A1-2019/133608
- CN-A- 111 248 994
- US-A1- 2007 083 239
- US-A1- 2010 023 004
- US-A1- 2021 177 503

## Description

### Field of the invention

**.** The present invention generally relates to an electronic apparatus for delivering Coherent Sine Burst Irreversible Electroporation energy to biological tissue.

### Background art

**.** Tissue ablation is used in numerous medical procedures to treat a patient. Ablation can be performed to remove or denature undesired tissue such as diseased cardiac cells. The ablation can be performed by passing energy, such as electrical energy, through one or more electrodes and causing tissue death where the electrodes are in contact. Ablation procedures can be performed on patients with any cardiac arrhythmia such as atrial fibrillation (AF) by ablating tissue in the heart.

**.** Radiofrequency ablation (RFA) is a medical procedure in which part of the electrical conduction system of the heart, tumour or other dysfunctional tissue is ablated using the heat generated from medium frequency alternating current, e.g. in the range of 350-500 kHz.

**.** Particularly, in this procedure an energy delivery device, such as a probe with or without a needle, is inserted into a target tissue to cause destruction of a target region of the cardiac tissue through the application of thermal energy. In fact, electrically induced thermal ablation such as RFA can be used to effectively and continuously locally ablate a tissue site as the energy delivery device is placed on the tissue surface. Although RFA can effectively ablate volumes of target tissue, there are limitations to this technique. One often cited problem using this procedure during cardiac ablation involves heat sink, a process whereby one aspect can include blood flow whereas the heat generated on the ablation element will be removed/dissipated by the cooler blood flows over the element. This heat dissipation effect can change both the shape and maximum volume of the tissue being ablated.

**.** More recently, to ablate cardiac or organ tissue, Pulsed Electric Fields (PEF) have been used as an alternative to the above-mentioned RFA. Pulsed Electric Fields (PEF) refer to application of intermittent, high-intensity electric fields for short periods of time (micro or nanoseconds), which results in cellular and tissue electroporation. Electroporation is a process whereby an applied electric field (i.e. PEF) results in the formation of pores in cell membranes. Pore formation leads to permeabilization, which can be reversible or irreversible, depending upon parameters of the applied PEF.

**.** In reversible electroporation, cells remain viable, and underlies the basis of electrochemotherapy and gene electrotransfer. In contrast, with irreversible electroporation (IRE), cells and tissue are non-viable because of programmed cell death cascade activation.

**.** IRE is a well-established treatment for solid tumours. However, IRE may also be useful in cardiology, particularly for cardiac ablation, given limitations of current thermal based approaches.

**.** When used to ablate cardiac tissue, IRreversible Electroporation (IRE) involves the application of electrical pulses to targeted tissue in the range of microseconds to milliseconds that can lead to non-thermally produced defects in the cell membrane that are nanoscale in size. These defects can lead to a disruption of homeostasis of the cell membrane, thereby causing irreversible cell membrane permeabilization which induces cell necrosis, without raising the temperature of the tissue ablation zone.

**.** Typical PEF parameters to cause IRE include 10-90 pulses, with a pulse length of microseconds or nanoseconds (usually 100 µs), at a frequency of 1-10 Hz, and with an electric field between 500 and 3000 V/cm. See reference Elad Maor et al "Pulsed electric fields for cardiac ablation and beyond: A state-of-the-art review", Heart Rhythm 2019;16:1112-1120).

**.** One challenge in designing PEF protocols is separating the effects electric fields can have on biological tissues. An electric field applied across tissue will induce heat by Joule's first law, which states power of heating is proportional to resistance and square of the current. To overcome this and to solely obtain electroporation damage, most PEF protocols use ultrashort pulses (microseconds) at low frequency (1-10 Hz). This balances heating with the cooling effect of physiological heat conduction and convection, preventing significant rises in temperature.

**.** IRE performed with unipolar electrical pulses has the disadvantage of causing intense muscle contractions. Therefore, clinical applications of IRE require the administration of general anaesthesia and neuroparalytic agents in order to eliminate the discomfort caused by muscle contractions seen during each pulse. However, receiving paralytic agents is undesirable for patients, and may deter them from seeking an electroporation based therapy.

**.** Electronic systems are known in the art for delivering IRreversible Electroporation (IRE) energy to a biological tissue, wherein these electronic systems are configured to generate high-frequency, bipolar waveforms for mitigating muscle contractions during electroporation based therapies.

**.** In particular, High-Frequency IRE (H-FIRE) is a technique for non-thermal tissue ablation that eliminates muscle contractions seen in IRE treatments performed with unipolar electric pulses. In fact, no visual or tactile evidence of muscle contraction was seen during H-FIRE at 250 kHz or 500 kHz. Therefore, H-FIRE can be performed clinically without the administration of paralytic agents.

**.** H-FIRE can involve the application of square wave electrical signals. For this purpose, it is known, for example, to use square wave signals centred at 500 kHz.

**.** However, rectangular waveforms comprise signal components having various frequencies and amplitudes that could create dangerous effects when IRE is used to specifically treat cardiac tissue. For example, a traditional IRE square wave signal has a 150Hz component at a voltage of around 6V, which poses a significant risk of heart stimulation.

**.** The issue with square wave pulsed electric fields are the similarities to that of an ICD (Internal Cardiac Defibrillator): these types of devices cause significant heart tissue damage when discharged. Using square wave pulsed electric field can cause heart tissue damage outside the desired zone. As such, sedation is required and square-wave delivery has to be synchronized with the R-wave of an ECG.

US2010/023004 A1 discloses electroporation systems and methods for reducing excessive heat generation in which pulsed high voltage energy is delivered to e.g. cardiac tissue.

**.** It is therefore still strongly felt the need of providing an Irreversible Electroporation (IRE) based treatment for a biological tissue, especially the heart tissue, which avoids cardiac muscle stimulation and does not require sedation of the patients.

### SUMMARY OF THE INVENTION

**.** It is the object of the present invention to provide an electronic apparatus for delivering Irreversible Electroporation (IRE) energy, particularly Coherent Sine Burst IRE, to a biological tissue to be treated, particularly a cardiac tissue, having structural and functional features such as to meet the aforementioned needs and overcome the drawbacks mentioned above with reference to the electronic systems of the prior art used for the same purpose.

**.** These and other objects are achieved by an electronic apparatus according to claim 1.

**.** Particularly, according to a first aspect of the invention there is provided an electronic apparatus for delivering Coherent Sine Burst Irreversible Electroporation energy, or IRE, to a biological tissue to be treated, particularly a cardiac tissue.

**.** The electronic apparatus comprises:
at least one electrode positionable either on or near the biological tissue to be treated; and
a power generator for supplying electric energy to the or each of the electrode.

**.** The power generator is configured to generate a respective electric signal to energize the or each electrode.

**.** The electric signal is formed by alternating over time a first electric signal with a second electric signal, each first electric signal being the same. The first electric signal is supplied to an individual electrode during a first time interval and the second electric signal is supplied to the individual electrode during a second time interval, subsequent to the first time interval. The first electric signal is a continuous bipolar signal comprising two or more basic sine waves in said first time interval, each basic sine wave having a peak-to-peak mean amplitude in the range 2,000 V to 20,000 v and consisting of one positive half-wave and one negative half-wave, each have wave having the same width. The first electric signal has a frequency in the range 25 kHz to 75 kHz. The second electric signal has an amplitude equal to zero in said second time interval.

**.** Applicant has verified that the electric signal S, generated by alternating over time the first electric signal, comprising two or more basic sine waves, with the second electric signal, having amplitude equal to zero, does not generate cardiac muscle stimulation. Therefore, in a first aspect, patients do not have to be sedated. In a further aspect, the delivery of energy does not have to be synchronized with the hearts R-wave of an ECG.

**.** Additionally, providing a first electric signal with a frequency in the range 25 kHz to 75 kHz desirably imparts sufficient energy to, as needed, cause irreversible tissue ablation, while continuing to avoid said cardiac muscle stimulation.

**.** Preferably said first electric signal has a frequency in the range of 35 kHz to 65 kHz.

**.** The first electric signal may have a frequency in the range of 40 kHz to 60 kHz.

**.** Optionally the first electric signal has a frequency in the range of 45 kHz to 55 kHz.

**.** Such frequency ranges advantageously deliver sufficient energy while avoiding cardiac muscle stimulation.

**.** Preferably the first electric signal comprises from two to fifteen basic sine waves in said first time interval.

**.** Optionally the second time interval of the second signal has a duration from at least 1 milli-second to 1 second.

**.** Such features desirably cause irreversible ablation while keeping any associated generation of thermal energy to a minimum.

**.** The electronic apparatus according to the invention further comprises a plurality of electrodes, and the power generator comprises a single control unit and a power unit for generating said respective electric signals, and the power unit is electrically connected to all electrodes of said plurality of electrodes whereby the plurality of electrodes provides one of a unipolar voltage, a bipolar voltage, or a combination of bipolar and unipolar voltage.

**.** Such an arrangement provides the option of achieving irreversible ablation relatively deep into a biological tissue, e.g. by having the plurality of electrodes provide a unipolar voltage, as well as shallower and more localised irreversible ablation within such tissue, e.g. by having the plurality of electrodes provide a bipolar voltage. In addition, having the electrodes provide a combination of bipolar and unipolar voltage provides the option of achieving relatively deep irreversible ablation, akin to that achieved with a unipolar voltage alone, but with less muscle stimulation than arises with solely a unipolar voltage of the same magnitude.

**.** Moreover, such irreversible ablation is achieved through the generation of voltage fields, i.e. extending away from the electrodes in the case of a unipolar voltage and extending between adjacent electrodes in the case of a bipolar voltage (or a combination of such voltage fields in the case of a combination of unipolar and bipolar voltages) which cause electroporation, i.e. cause an increase in the permeability of cell membranes within a biological tissue, and so avoids the generation of heat within such tissue as would be the case, e.g. if a current was instead driven through the tissue.

**.** Optionally the power unit is configured, in the presence of an electrical return path, to supply each electrode with the same individual electric signal in order to cause the electrodes to provide a unipolar voltage via the electrical return path.

**.** The power unit may be configured, in the absence of an electrical return path, to supply adjacent electrodes with individual electric signals that are 180° out of phase with one another in order to cause the electrodes to provide a bipolar voltage.

**.** Such arrangements beneficially provide the required unipolar voltage, or bipolar voltage, in a readily implementable manner.

**.** In the invention the power unit is configured, in the presence of an electrical return path, to supply adjacent electrodes with individual electric signals that are out of phase with one another in order to cause the electrodes to provide a combination of bipolar and unipolar voltage.

**.** Having the electrodes provide a combination of bipolar and unipolar voltage in such a way, i.e. in the presence of an electrical return path, means that most electrical return takes place between adjacent electrodes, while some does still take place via the electrical return path, i.e. externally, and so is a practical way of helping to achieve relatively deep irreversible ablation, akin to that achieved with a unipolar voltage alone, but with less muscle stimulation than arises with solely a unipolar voltage of the same magnitude.

**.** In the invention the power unit is configured to vary the extent to which the individual electric signals supplied to adjacent electrodes are out of phase with one another.

**.** Preferably the power unit is configured to vary the extent to which the individual electric signals supplied to adjacent electrodes are out of phase with one another between 0° and 180°.

**.** Such varying of the extent to which the individual electric signals supplied to adjacent electrodes are out of phase with one another desirably allows the depth of irreversible ablation to be altered.

**.** According to a second aspect, not part of the claimed invention as such but useful for the general understanding of the invention, there is provided a method for controlling at least one electrode in an electronic apparatus for delivering Coherent Sine Burst Irreversible Electroporation energy to a biological tissue to be treated, the electronic apparatus comprising at least one electrode positionable either on or near the biological tissue to be treated, and a power generator for supplying electric energy to the or each electrode, the power generator being configured to generate a respective electric signal to energize the or each individual electrode; and the method comprising the steps of:
forming the or each electric signal (S) by alternating over time a first electric signal (S1) with a second electric signal (S2), each first electric signal (S1) being the same;
supplying to an individual electrode (3) the first electric signal (S1) during a first time interval (T1) and the second electric signal (S2) during a second time interval (T2), subsequent to the first time interval (T1),
said first electric signal (S1) being a continuous bipolar signal comprising two or more basic sine waves (SB) in the first time interval (T1), each basic sine wave having a peak-to-peak mean amplitude in the range 2,000 V to 20,000 V and consisting of one positive half-wave and one negative half-wave, each half-wave having the same width, and the first electric signal (S1) having a frequency in the range 25 kHz to 75 kHz, and
said second electric signal (S2) having an amplitude equal to zero in a second time interval (T2) subsequent to the first time interval (T1).

**.** According to a second aspect, not part of the claimed invention as such but useful for the general understanding of the invention, there is provided a method of ablating biological tissue in a patient using at least one electrode of an electronic apparatus for delivering Coherent Sine Burst Irreversible Electroporation energy to the biological tissue to be treated, the electronic apparatus comprising at least one electrode, and a power generator for supplying electric energy to the or each electrode, the power generator being configured to generate a respective electric signal to energize the or each individual electrode; and
the method comprising the steps of:
positioning the or each electrode (3) on or near the biological tissue (1) to be treated;
forming the or each electric signal (S) by alternating over time a first electric signal (S1) with a second electric signal (S2), each first electric signal (S1) being the same;
supplying to an individual electrode (3) the first electric signal (S1) during a first time interval (T1) and the second electric signal (S2) during a second time interval (T2), subsequent to the first time interval (T1),
said first electric signal (S1) being a continuous bipolar signal comprising two or more basic sine waves (SB) in the first time interval (T1), each basic sine wave having a peak-to-peak mean amplitude in the range 2,000 V to 20,000 V and consisting of one positive half-wave and one negative half-wave, each half-wave having the same width, and the first electric signal (S1) having a frequency in the range 25 kHz to 75 kHz, and
said second electric signal (S2) having an amplitude equal to zero in a second time interval (T2) subsequent to the first time interval (T1).

**.** The methods share the benefits associated with the corresponding features of the electronic apparatus of the invention.

### Drawings

**.** Further features and advantages of the invention will become apparent from the description provided below of exemplary embodiment thereof, given by way of nonlimiting example, with reference to the accompanying drawings, in which:
- **Figure 1** shows schematically an electronic apparatus for delivering Coherent Sine Burst Irreversible Electroporation energy, or IRE, to a biological tissue according to a first embodiment of the invention, wherein the electronic apparatus comprises a plurality of electrodes positionable either on or near the biological tissue to be treated, and a power generator for supplying electric energy to each of the electrodes;
- **Figure 2** shows, with a block diagram, the power generator of the electronic apparatus of figure 1 comprising a single control unit and a power unit;
- **Figure 3** shows, with a diagram as a function of time, an example of electric signal generated by the power generator of figure 1 by alternating over time a first electric signal with a second electric signal;
- **Figure 4** shows at a higher resolution a portion of the diagram of figure 3 representing the first electric signal;
- **Figure 5** illustrates a Fourier Analysis of the first electric signal including eleven basic sine waves at 50KHz;
- **Figure 6** shows schematically the electronic apparatus according to the first embodiment of the invention, wherein the power generator is configured to supply adjacent electrodes, respectively, with sine-wave electrical signals "in phase" such that the electrodes provide a unipolar voltage;
- **Figure 7** illustrates the electric field generated by the Figure 6 arrangement;
- **Figure 8** shows schematically the electronic apparatus according to the first embodiment of the invention, wherein the power generator is configured to supply adjacent electrodes, respectively, with sine-wave electrical signals "out of phase" such that the electrodes provide a bipolar voltage;
- **Figure 9** illustrates the electric field generated by the Figure 8 arrangement;
- **Figure 10** shows schematically the electronic apparatus according to the first embodiment of the invention, wherein the power generator is configured, in the presence of an electrical return path to supply adjacent electrodes, respectively, with sine-wave electrical signals "out of phase" such that the electrodes provide a combination of bipolar and unipolar voltage.
- **Figure 11** illustrates the electric field generated by the Figure 10 arrangement.

**.** The same or similar elements are indicated in the drawings by the same reference numeral.

### Description of some preferred embodiments

**.** The present invention can be understood more readily by reference to the following detailed description, examples, drawing, and their previous and following description. However, before the present devices, systems, and/or methods are disclosed and described, it is to be understood that this invention is not limited to the specific devices, systems, and/or methods disclosed unless otherwise specified, as such can, of course, vary, but is defined in the appended claims. It is also to be understood that the terminology used herein is for the purpose of describing particular aspects only and is not intended to be limiting.

**.** The following description of the invention is provided as an enabling teaching of the invention in its best, currently known embodiment. To this end, those skilled in the relevant art will recognize and appreciate that many changes can be made to the various aspects of the invention described herein, while still obtaining the beneficial results of the present invention. It will also be apparent that some of the desired benefits of the present invention can be obtained by selecting some of the features of the present invention without utilizing other features. Accordingly, those who work in the art will recognize that many modifications and adaptations to the present invention are possible and can even be desirable in certain circumstances and are a part of the present invention, as defined in the appended claims.

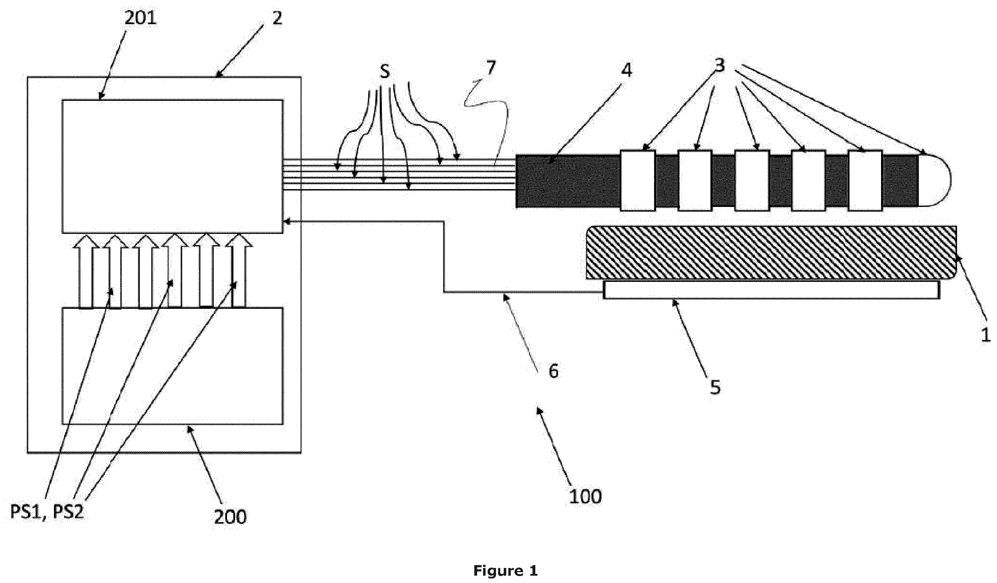

**.** In accordance with a general embodiment, with reference to figure 1, an electronic apparatus for delivering Coherent Sine Burst Irreversible Electroporation energy, or IRE, to a biological tissue 1 to be treated according to the present invention is globally denoted by reference numeral 100.

**.** The electronic apparatus 100 comprises a plurality of electrodes 3 positionable either on or near the biological tissue 1 to be treated and a power generator 2 for supplying electric energy to each of the electrodes 3. More particularly, six electrodes 3 are shown in the example of figure 1, each of which is operatively associated to a catheter 4. Other embodiments of the invention may include fewer than or more than six electrodes.

**.** In a preferred embodiment, the biological tissue 1 to be treated is a cardiac tissue.

**.** The power generator 2 is electrically connected to the electrodes 3, particularly with six wires 7, and is configured to generate a respective electric signal S to energize each individual electrode 3, i.e. such that the electrodes together apply voltage electric fields to the biological tissue 1.

**.** In addition, the electronic apparatus 100 comprises a further electrode 5 acting as a return electrode, i.e. an electrical return path, for the voltage electrical fields applied to the biological tissue 1. More particularly, this return electrode 5 or backplate is electrically connected to the power generator 2 through a respective return wire 6.

**.** In an alternative embodiment (not shown in the figures) the electronic apparatus 100 can comprise a single electrode 3 positionable either on or near the biological tissue 1 to be treated. The power generator 2 is electrically connected to this single electrode 3 and is configured to generate an electric signal S to energize said electrode 3, i.e. to apply a voltage electric field to the biological tissue 1 through the single electrode 3.

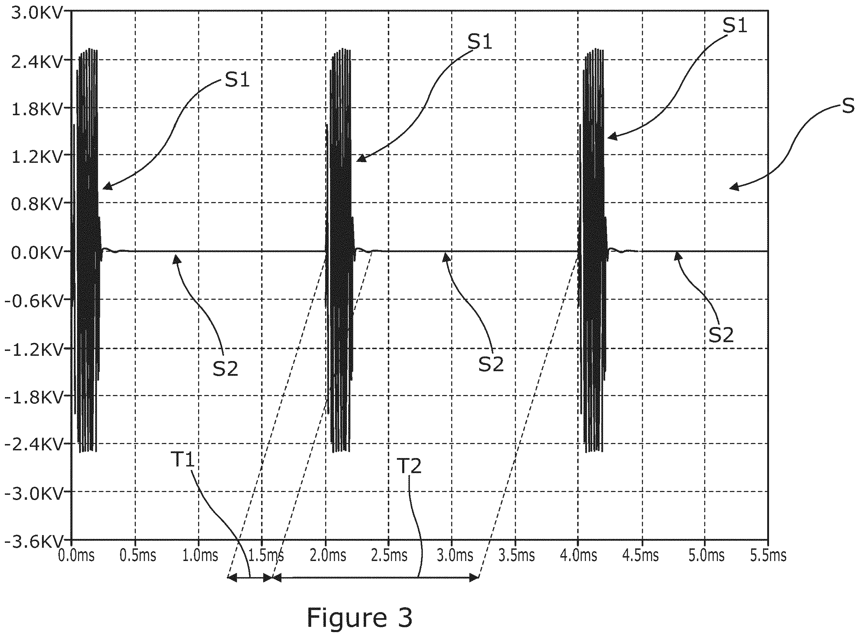

**.** In more detail, with reference to figures 3-4, the electric signal S is formed by alternating over time a first electric signal S1 with a second electric signal S2. As shown in Figure 3, each first electric signal S1 is the same. The first electric signal S1 is supplied to an individual electrode 3 during a first time interval T1 and the second electric signal S2 is supplied to the same individual electrode 3 during a second time interval T2 subsequent to the first time interval T1.

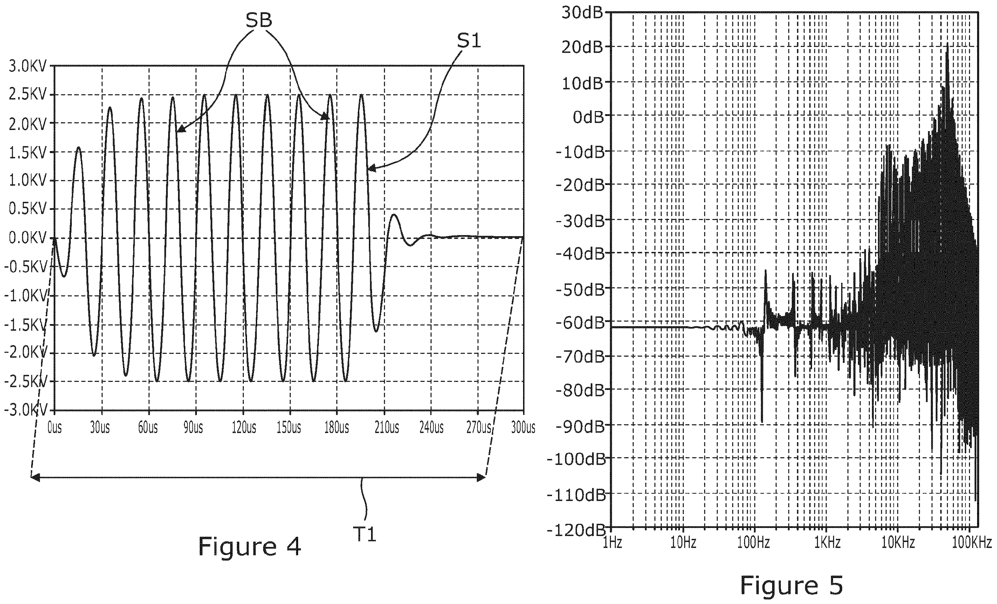

**.** The first electric signal S1 is a continuous bipolar signal comprising two or more basic sine waves SB in said first time interval T1. Each basic sine wave consisting in one positive half-wave and one negative half-wave, with each half-wave having the same width, as shown for example in Figure 4. The second electric signal S2 has an amplitude equal to zero in said second time interval T2.

**.** For example, the electric signal S depicted in figure 3 comprises three first electric signals S1, wherein two of said first electric signals S1 are spaced from one another by the second electric signal S2 which is null in the second time interval T2.

**.** In an alternative embodiment, the first electric signal S1 comprises two or more basic sine waves SB repeated over said first time interval T1.

**.** With the present invention, the Applicant proposes the use of an electric signal S for ablating the tissue 1 that consists of several sine wave bursts, i.e. a plurality of first electric signals S1, spaced by cooling periods, i.e. the second electric signals S2, in which no electrical energy is transferred to the electrodes 3. The sine wave bursts are created in a way that avoids stimulation of the heart, even if applied for longer periods of time. This is obtained by choosing the waveforms such that the net voltage of harmonic waves created in a region between 1 Hz and 200 Hz is negligible. In fact, the frequency interval 1 Hz and 200 Hz has to be avoided in order to avoid heart stimulation.

**.** In more detail, the diagram of electric signal S shown in figure 3 includes three sine wave bursts, each spaced by a cooling period of 1.5 msec.

**.** In figure 5, a Fourier Analysis diagram of the first electric signal S1 including eleven basic sine waves SB, for example at 50 KHz is depicted. Particularly, this Fourier Analysis diagram indicates that the voltage amplitude of the 50 kHz signal component, i.e. the desired component, is 67 dB higher than the 150 Hz signal component, which is the harmful component. In particular, a peak voltage of 1.5 kV for the signal component at 50 kHz corresponds about a peak voltage of 0.0005 V for the signal component at 150 Hz, i.e. a peak voltage near to zero which does not create heart stimulation.

**.** In comparison, a traditional IRE square wave signal has a 150 Hz signal component at a voltage of around 6 V, which would create a significant risk of heart stimulation.

**.** To that end, in the first embodiment shown the first electric signal S1 has a frequency in the range of 25 kHz - 75 kHz.

**.** Preferably the first electric signal S1 has a frequency in the range of 35 kHz to 65 kHz, more preferably 40 kHz to 60 kHz, and most preferably 45 kHz to 55 kHz. More particularly still, the first electric signal S1 may have a frequency of 50 kHz.

**.** The first electric signal S1 may comprise from two to fifteen basic sine waves SB in said first time interval T1.

**.** In fact, depending on tissue ablation requirements, when a voltage applied to the tissue 1 with the electric signal S is selected to be higher, the generator 2 provides the first electric signal S1 including fewer basic sine waves SB repeated over the first time interval T1, i.e. fewer cycles of the first S1 and second S2 electric signals are required. On the contrary, when a voltage applied to the tissue 1 with the electric signal S is selected to be lower, the generator 2 provides the first electric signal S1 including more basic sine waves SB repeated over said first time interval, i.e. the more the number of cycles of the first S1 and second S2 electric signals are required. Therefore, the voltage applied to the tissue 1 with the electric signal S can be obtained as a trade off between the number of cycles and the number basic sine waves SB repeated over said first time interval T1.

**.** The second time interval T2 of the second signal S2, i.e. the cooling period, has a duration from at least 1 milli-second to 1 second.

**.** In particular, the duration of the second time interval T2 can be changed based on requirements for heat dissipations.

**.** The peak-to-peak mean amplitude of each basic sine wave SB is in the range of 2,000 V to 20,000 V. The preferred amount is tissue dependent. In particular, upon power up of the power generator 2, the initial setting could be -2.5 kV and +2.5 kV before changing to accommodate the various tissue requirements.

**.** In general, according to the present invention, properties of the electric signal S can be changed as long as the amplitude of signal components below 200 Hz are negligible.

**.** Meanwhile, the power generator 2 comprises a single control unit 200 and a power unit 201 for generating the electric signal S. Particularly, the power unit 201 is electrically connected to all electrodes 3 of said plurality of electrodes. In this manner the plurality of electrodes 3 is able to provide one of (i) a unipolar voltage; (ii) a bipolar voltage; or (iii) a combination of bipolar and unipolar voltage.

**.** More particularly, the power unit 201 comprises a power module 202 driven by the single control unit 200 to generate said first electric signal S1 during the first time interval T1 and to generate said second electric signal S2 during the second time interval T2.

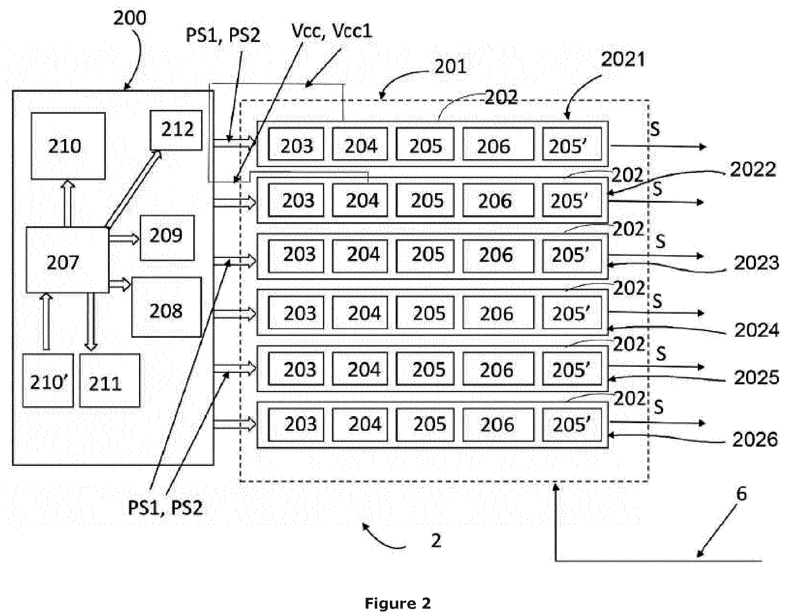

**.** By way of example, as shown schematically in Figure 2, the power module 202 comprises one or more of:
- a drive circuit block 203 controlled by the single control unit 200 for generating said first S1 or second S2 electric signal starting from drive signals PS1, PS2 provided by the single control unit 200 or a signal null, respectively;
- a selecting block 204 selectively controlled by said drive circuit block 203 to change continuously the electric energy level associated to said electric signal S; and
- a filtering and electrical isolation block 205, 206, 205'.

**.** In the example of figure 2, the power unit 201 comprises six power modules 202. Each power module 202 is configured to generate and supply a respective electric signal S to one, individual electrode 3. More particularly, with reference to figure 2, from top to bottom, the power unit 201 includes a first power module 2021, a second power module 2022, a third power module 2023, a fourth power module 2024, a fifth power module 2025, a sixth power module 2026. The returns of all power modules 2021-2026 are connected together and correspond to the return wire 6, i.e. a single electrical return path.

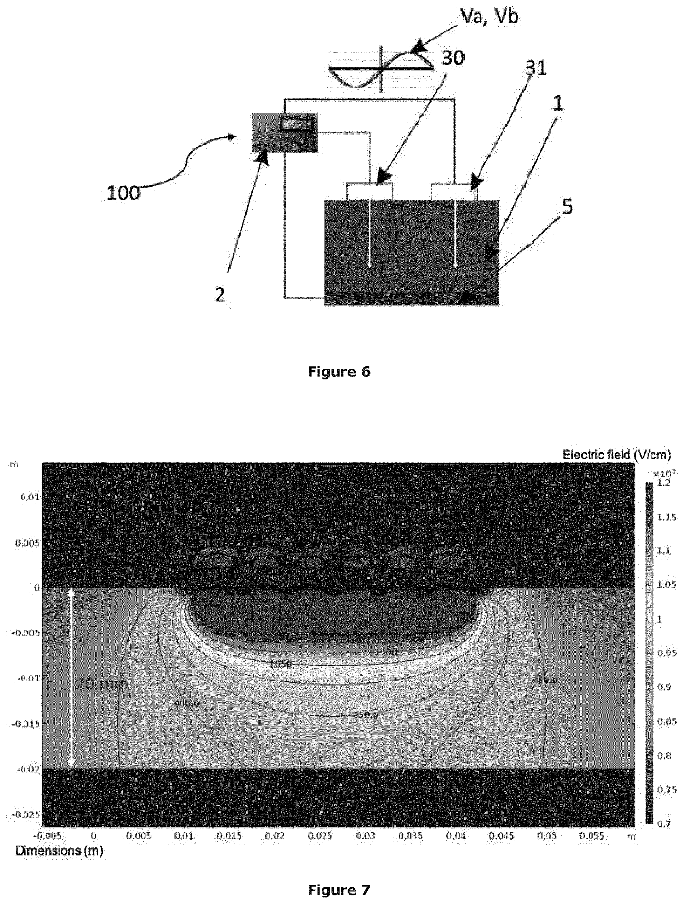

**.** With reference to Figure 6, the power unit 201 is configured, in the presence of an electrical return path, i.e. the return electrode 5 and associated return wire 6, to supply each electrode 3 with the same individual electric signal S, i.e. the same waveforms in phase with one another, in order to cause the electrodes 3 to provide a unipolar voltage via the electrical return path.

**.** More particularly, the power generator 2 is configured to supply first and second adjacent electrodes 30, 31, respectively, with the same sine-wave electrical signals Va, Vb, which are "in phase" with one another. Respective power modules, e.g. the first and second power modules 2021, 2022, supply a given first or second electrode 30, 31 with the said same sine-wave electrical signal Va, Vb. In this manner the power generator 2 causes the electrodes 30, 31 to deliver a unipolar voltage for irreversible electroporation to the tissue 1, with the resulting power being driven by the difference in voltage between the first and second electrodes 30, 31 and the ground potential (0 V) associated with the return electrode 5. In this case, current flows from the first and second electrodes 30, 31 to ground, i.e. to the return electrode 5, and there is no voltage difference between the first and second electrodes at any moment in time - thus no bipolar current flow between such electrodes.

**.** As the voltage oscillates between positive and negative peaks, the current moves to and from the ablation site and the return electrode 5, with an example of the resulting electric field that develops between the electrodes 3 and the return electrode 5 being shown in Figure 7. Relatively deep ablation is possible using such a unipolar voltage, e.g. up to about 20mm in depth.

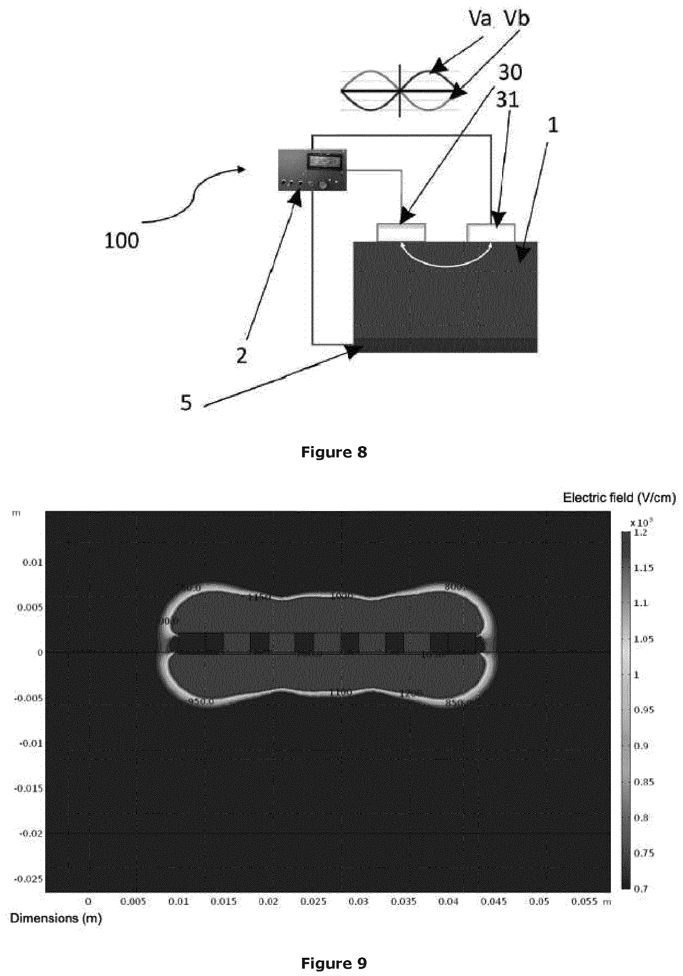

**.** With reference to Figure 8, the power unit 201 is also configured, in the absence of an electrical return path, i.e. with the return electrode 5 and/or associated return wire 6 disconnected, to supply adjacent electrodes 3 with individual electric signals S that are 180° out of phase with one another in order to cause the electrodes 3 to provide a bipolar voltage.

**.** More particularly, the power generator 2 is configured to supply the first electrode 30 with a first sine-wave electrical signal Va, and to supply the second, adjacent electrode 31 with a second sine-wave electrical signals Vb which is "out of phase" with the first sine-wave electrical signal Va, and more particularly still has a phase difference of 180° compared to the first sine-wave electrical signal Va. Respective power modules supply the first and second sine-wave electrical signals Va, Vb, e.g. the first power module 2021 supplies the first electrode 30 with the first sine-wave electrical signal Va and the second power module 2022 supplies the second electrode 31 with the second sine-wave electrical signal Vb.

**.** In this case, a voltage is applied only between each of the first 30 and the second 31 electrodes and the resulting electric field that develops is, as shown by way of example in Figure 9, relatively shallow and localised, e.g. inside a heart wall or blood pool. Such shallow and localised electric field also helps to minimise muscle stimulation. Accordingly, in this manner, i.e. using a bipolar voltage, localised ablation up to a depth of at least 5mm, and possibly as much as 8mm, is possible.

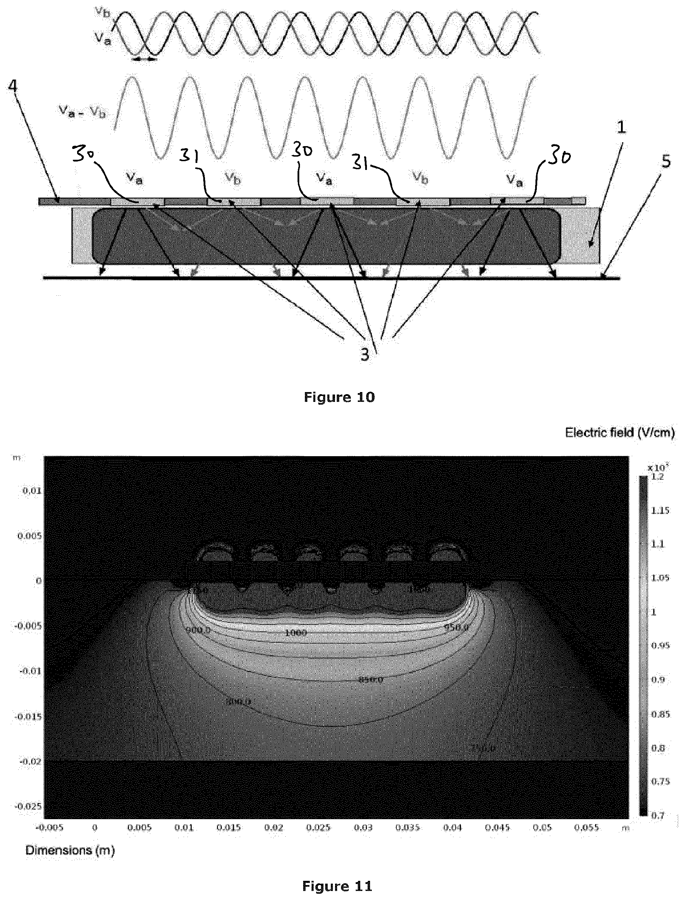

**.** In addition to the foregoing, with reference to Figure 10, the power unit 201 is also configured, in the presence of an electrical return path, i.e. with the return electrode 5 and associated return wire 6 connected, to supply adjacent electrodes 3 with individual electric signals S that are out of phase with one another in order to cause the electrodes 3 to provide a combination of bipolar and unipolar voltage.

**.** More particularly, a first electrode 30 in a pair of adjacent electrodes 3 is provided with a first sine-wave electric signal Va and a second electrode 31 in the said pair of adjacent electrodes 3 is provided with a second sine-wave electric signal Vb. The electrical return is mostly between adjacent electrodes 30, 31, such that a bipolar voltage equal in magnitude to Va - Vb arises between such adjacent electrodes 30, 31, but an electrical return component does arise via the return electrode 5 and associated return wire 6, such that a first unipolar voltage equal in magnitude to Va arises between each first electrode 30 and the return electrode 5 and a second unipolar voltage equal in magnitude to Vb arises between each second electrode 31 and the return electrode 5.

**.** The resulting electrical field is shown by way of example in Figure 11, which permits relatively deep irreversible ablation, e.g. akin to that achieved with a unipolar voltage alone, but with less muscle stimulation than arises with solely a unipolar voltage of the same magnitude.

**.** Additionally, the power unit 201 can vary the extent to which the individual electric signals S, i.e. the first and second sine-wave electric signals, Va, Vb, supplied to adjacent electrodes 3 are out of phase with one another. More particularly, the power unit 201, i.e. individual power modules 202 therein, can vary the extent to which the individual electric signals S, i.e. the first and second sine-wave electric signals, Va, Vb, supplied to adjacent electrodes 3 are out of phase with one another between 0° and 180°.

**.** When the first and second sine-wave electric signals, Va, Vb supplied to adjacent electrodes 3 are out of phase with one another by 0°, i.e. the are in effect *in-phase* with one another, the electrodes only provide a unipolar voltage, of respective identical magnitudes Va and Vb. Meanwhile, when the first and second sine-wave electric signals, Va, Vb supplied to adjacent electrodes 3 are out of phase with one another by 180° the electrodes only provide a bipolar voltage, of magnitude Va - Vb.

**.** In contrast, when the first and second sine-wave electric signals, Va, Vb supplied to adjacent electrodes 3 are out of phase with one another by an amount between 0° and 180° the ratio of unipolar voltage to bipolar voltage varies depending on the phase difference. For example, when the phase difference is 60° the magnitude of bipolar voltage generated between two adjacent electrodes 30, 31 is the same as the magnitude of the unipolar voltage generated between each electrode 30, 31 and the return electrode 5, and when the phase difference is 90° the magnitude of bipolar voltage generated between two adjacent electrodes 30, 31 is 1.41 (i.e. √2) times greater than the magnitude of the unipolar voltage generated between each electrode 30, 31 and the return electrode 5. In this way, i.e. by varying the ratio of deeper penetrating unipolar voltage to less penetrating bipolar voltage, it is possible to modify the depth of irreversible ablation achieved. Moreover, utilising such a combination of unipolar and bipolar voltage results in less muscle stimulation than arises with the application of a solely unipolar voltage of the same unipolar magnitude. In other words, the presence also of a bipolar voltage has a mitigating effect in terms of reducing muscle stimulation.

**.** In other embodiments of the invention (not shown), the power generator, i.e. power unit, may be configured to alternately cause the electrodes to provide a unipolar voltage and a bipolar voltage, for example by time division multiplexing. In such embodiments the power unit may, as a first step cause the electrodes to provide a unipolar voltage, i.e. by applying a voltage between each electrode and the return electrode, and then, as a second step cause the electrodes to provide a bipolar voltage, i.e. by applying a voltage between adjacent electrodes. Such first and second steps preferably are then followed by an off period.

**.** According to such other embodiments, by choosing a different combination of unipolar and bipolar voltages, e.g. by multiplexing, the ratio of bipolar to unipolar voltage can similarly be varied, e.g. from 4 to 1 to all unipolar, and thereby again vary the depth of ablation achieved.

### LIST OF REFERENCE NUMERALS

- 100: Electronic apparatus
- 1: biological tissue
- 2: power generator
- 3: electrodes
- 30: first electrode
- 31: second electrode
- 4: catheter
- 5: further electrode, return electrode
- 6: return wire
- 7: wire
- S: electric signal
- S1: first electric signal
- S2: second electric signal
- T1: first time interval
- T2: second time interval
- SB: basic sine wave
- 200: single control unit
- 201: power unit
- 202: power module
- 203: drive circuit block - amplifier circuit in emitter Follower configuration
- 204: selecting block - H-Bridge circuit - power inverter block
- 205: first series resonance filter
- 206: electrical isolation block -transformer
- 205': second series resonance filter
- 207: Microprocessor
- 208: variable High Voltage Power Supply block
- 209: Programmable Logic Controller block
- 210: Video interface block
- 210': Push Button block
- 211: Watch Dog block
- 212: Audio interface block
- 230: first transformer circuit
- Vcc: supply voltage signal - first direct current power supply voltage
- Vcc1: supply voltage signal - second direct current power supply voltage
- PS1: first pulsed signal
- PS2: second pulsed signal
- Va, Vb: sine-wave electrical signals
- 2021: first power module
- 2022: second power module
- 2023: third power module
- 2024: fourth power module
- 2025: fifth power module
- 2026: sixth power module.

## Claims

1. Electronic apparatus (100) for delivering Coherent Sine Burst Irreversible Electroporation energy to a biological tissue (1) to be treated, the electronic apparatus comprising:
at least one electrode (3) positionable either on or near the biological tissue (1) to be treated; and
a power generator (2) for supplying electric energy to the or each electrode (3), said power generator (2) being configured to generate a respective electric signal (S) to energize the or each individual electrode (3),
wherein the or each electric signal (S) is formed by alternating over time a first electric signal (S1) with a second electric signal (S2), each first electric signal (S1) being the same; said first electric signal (S1) is supplied to an individual electrode (3) during a first time interval (T1) and said second electric signal (S2) is supplied to the individual electrode (3) during a second time interval (T2) subsequent to the first time interval (T1);
said first electric signal (S1) is a continuous bipolar signal comprising two or more basic sine waves (SB) in said first time interval (TI), each basic sine wave having a peak-to-peak mean amplitude in the range 2,000 V to 20,000 V and consisting of one positive half-wave and one negative half-wave, each half-wave having the same width, and the first electric signal (SI) having a frequency in the range 25 kHz to 75 kHz; and
said second electric signal (S2) has an amplitude equal to zero in said second time interval (T2), the electronic apparatus (100) further comprising a plurality of electrodes (3), wherein said power generator (2) comprises a single control unit (200) and a power unit (201) for generating said respective electric signals (S);
said power unit (201) being electrically connected to all electrodes (3) of said plurality of electrodes whereby the plurality of electrodes provides one of a unipolar voltage, a bipolar voltage, or a combination of bipolar and unipolar voltage;
wherein the power unit (201) is configured, in the presence of an electrical return path, to supply adjacent electrodes (3) with individual electric signals (S) that are out of phase with one another in order to cause the electrodes (3) to provide a combination of bipolar and unipolar voltage; wherein the power unit (201) is configured to vary the extent to which the individual electric signals (S) supplied to adjacent electrodes (3) are out of phase with one another.

2. Electronic apparatus (100) according to claim 1, wherein said first electric signal (S1) has a frequency in the range of 35 kHz to 65 kHz.

3. Electronic apparatus (100) according to claim 2, wherein said first electric signal (S1) has a frequency in the range of 40 kHz to 60 kHz.

4. Electronic apparatus (100) according to claim 3, wherein said first electric signal (S1) has a frequency in the range of 45 kHz to 55 kHz.

5. Electronic apparatus (100) according to any preceding claim, wherein said first electric signal (S1) comprises from two to fifteen basic sine waves (SB) in said first time interval (T1).

6. Electronic apparatus (100) according to claim 1, wherein the second time interval (T2) of the second signal (S2) has a duration from at least 1 millisecond to 1 second.

7. Electronic apparatus (100) according to Claim 1 wherein the power unit (201) is configured to vary the extent to which the individual electric signals (S) supplied to adjacent electrodes (3) are out of phase with one another between 0° and 180°.

## Patentansprüche

1. Elektronisches Gerät (100) zur Abgabe von Coherent Sine Burst Irreversible Electroporation - Energie an ein zu behandelndes biologisches Gewebe (1), wobei das elektronische Gerät umfasst:
mindestens eine Elektrode (3), die entweder auf oder in der Nähe des zu behandelnden biologischen Gewebes (1) positioniert werden kann; und
einen Stromgenerator (2) zur Versorgung der oder jeder Elektrode (3) mit elektrischer Energie, wobei der Stromgenerator (2) so konfiguriert ist, dass er ein entsprechendes elektrisches Signal (S) erzeugt, um die oder jede einzelne Elektrode (3) mit elektrischer Energie zu versorgen ,
wobei das oder jedes elektrische Signal (S) durch zeitliches Abwechseln eines ersten elektrischen Signals (S1) mit einem zweiten elektrischen Signal (S2) gebildet wird, wobei jedes erste elektrische Signal (S1) das gleiche ist; das erste elektrische Signal (S1) einer einzelnen Elektrode (3) während eines ersten Zeitintervalls (T1) zugeführt wird und das zweite elektrische Signal (S2) der einzelnen Elektrode (3) während eines zweiten Zeitintervalls (T2) nach dem ersten Zeitintervall (T1) zugeführt wird;
das erste elektrische Signal (S1) ein kontinuierliches bipolares Signal ist, das zwei oder mehr Basis-Sinuswellen (SB) in dem ersten Zeitintervall (TI) umfasst, wobei jede Basis-Sinuswelle eine mittlere Spitze-Spitze-Amplitude im Bereich von 2.000 V bis 20.000 V aufweist und aus einer positiven Halbwelle und einer negativen Halbwelle besteht, wobei jede Halbwelle die gleiche Breite aufweist, und das erste elektrische Signal (SI) eine Frequenz im Bereich von 25 kHz bis 75 kHz aufweist; und
das zweite elektrische Signal (S2) eine Amplitude gleich Null in dem zweiten Zeitintervall (T2) aufweist, wobei das elektronische Gerät (100) ferner eine Vielzahl von Elektroden (3) umfasst, wobei der Stromgenerator (2) eine einzelne Steuereinheit (200) und eine Stromversorgungseinheit (201) zum Erzeugen der jeweiligen elektrischen Signale (S) umfasst;
wobei die Stromversorgungseinheit (201) elektrisch mit allen Elektroden (3) der Vielzahl von Elektroden verbunden ist, wobei die Vielzahl von Elektroden entweder eine unipolare Spannung, eine bipolare Spannung oder eine Kombination aus bipolarer und unipolarer Spannung bereitstellt;
wobei die Stromversorgungseinheit (201) so konfiguriert ist, dass sie bei Vorhandensein eines elektrischen Rückleitungspfads benachbarte Elektroden (3) mit einzelnen elektrischen Signalen (S) versorgt, die zueinander phasenverschoben sind, um zu bewirken, dass die Elektroden (3) eine Kombination bereitstellen aus
bipolarer und unipolarer Spannung; wobei die Stromversorgungseinheit (201) so konfiguriert ist, dass sie das Ausmaß variiert, in dem die einzelnen elektrischen Signale (S), welche benachbarten Elektroden (3) zugeführt werden, zueinander phasenverschoben sind.

2. Elektronisches Gerät (100) nach Anspruch 1, wobei das erste elektrische Signal (S1) eine Frequenz im Bereich von 35 kHz bis 65 kHz aufweist.

3. Elektronisches Gerät (100) nach Anspruch 2, wobei das erste elektrische Signal (S1) eine Frequenz im Bereich von 40 kHz bis 60 kHz aufweist.

4. Elektronisches Gerät (100) nach Anspruch 3, wobei das erste elektrische Signal (S1) eine Frequenz im Bereich von 45 kHz bis 55 kHz aufweist.

5. Elektronisches Gerät (100) nach einem der vorhergehenden Ansprüche, wobei das erste elektronische Signal (S1) zwei bis fünfzehn Basis-Sinuswellen (SB) in dem ersten Zeitintervall (T1) umfasst.

6. Elektronisches Gerät (100) nach Anspruch 1, wobei das zweite Zeitintervall (T2) des zweiten Signals (S2) eine Dauer von mindestens 1 Millisekunde bis 1 Sekunde aufweist.

7. Elektronisches Gerät (100) nach Anspruch 1, wobei die Stromversorgungseinheit (201) so konfiguriert ist, dass sie das Ausmaß, in dem die einzelnen elektrischen Signale (S), welche benachbarten Elektroden (3) zugeführt werden, zueinander phasenverschoben sind, zwischen 0° und 180° variiert.

## Revendications

1. Appareil électronique (100) pour délivrer de l'énergie d'électroporation irréversible à salve sinusoïdale cohérente à un tissu biologique (1) à traiter, l'appareil électronique comprenant :
au moins une électrode (3) pouvant être positionnée soit sur soit à proximité du tissu biologique (1) à traiter ; et
un générateur d'énergie (2) pour fournir de l'énergie électrique à la ou à chaque électrode (3), ledit générateur d'énergie (2) étant configuré pour générer un signal électrique (S) respectif pour exciter la ou chaque électrode (3) individuelle,
dans lequel le ou chaque signal électrique (S) est formé par alternance dans le temps d'un premier signal électrique (S1) avec un deuxième signal électrique (S2), chaque premier signal électrique (S1) étant le même ; ledit premier signal électrique (S1) est fourni à une électrode (3) individuelle pendant un premier intervalle de temps (T1) et ledit deuxième signal électrique (S2) est fourni à l'électrode (3) individuelle pendant un deuxième intervalle de temps (T2) subséquent au premier intervalle de temps (T1) ;
ledit premier signal électrique (S1) est un signal bipolaire continu comprenant deux ou plusieurs ondes sinusoïdales de base (SB) dans ledit premier intervalle de temps (T1), chaque onde sinusoïdale de base ayant une amplitude moyenne de crête à crête dans la plage de 2 000 V à 20 000 V et étant constituée d'une demi-onde positive et d'une demi-onde négative, chaque demi-onde ayant la même largeur, et le premier signal électrique (S1) ayant une fréquence dans la plage de 25 kHz à 75 kHz ; et
ledit deuxième signal électrique (S2) a une amplitude égale à zéro dans ledit deuxième intervalle de temps (T2), l'appareil électronique (100) comprenant en outre une pluralité d'électrodes (3), dans lequel ledit générateur d'énergie (2) comprend une seule unité de commande (200) et une unité d'énergie (201) pour générer lesdits signaux électriques (S) respectifs ;
ladite unité d'énergie (201) étant reliée électriquement à toutes les électrodes (3) de ladite pluralité d'électrodes moyennant quoi la pluralité d'électrodes fournit une parmi une tension unipolaire, une tension bipolaire ou une combinaison de tension bipolaire et unipolaire ;
dans lequel l'unité d'énergie (201) est configurée, en présence d'un chemin de retour électrique, pour alimenter des électrodes (3) adjacentes avec des signaux électriques (S) individuels qui sont déphasés les uns avec les autres afin d'amener les électrodes (3) à fournir une combinaison de tension bipolaire et unipolaire ; dans lequel l'unité d'énergie (201) est configurée pour faire varier la mesure dans laquelle les signaux électriques (S) individuels fournis à des électrodes (3) adjacentes sont déphasés les uns avec les autres.

2. Appareil électronique (100) selon la revendication 1, dans lequel ledit premier signal électrique (S1) a une fréquence dans la plage de 35 kHz à 65 kHz.

3. Appareil électronique (100) selon la revendication 2, dans lequel ledit premier signal électrique (S1) a une fréquence dans la plage de 40 kHz à 60 kHz.

4. Appareil électronique (100) selon la revendication 3, dans lequel ledit premier signal électrique (S1) a une fréquence dans la plage de 45 kHz à 55 kHz.

5. Appareil électronique (100) selon l'une quelconque revendication précédente, dans lequel ledit premier signal électrique (S1) comprend de deux à quinze ondes sinusoïdales de base (SB) dans ledit premier intervalle de temps (T1).

6. Appareil électronique (100) selon la revendication 1, dans lequel le deuxième intervalle de temps (T2) du deuxième signal (S2) a une durée d'au moins 1 milliseconde à 1 seconde.

7. Appareil électronique (100) selon la revendication **1,** dans lequel l'unité d'énergie (201) est configurée pour faire varier la mesure dans laquelle les signaux électriques (S) individuels fournis à des électrodes (3) adjacentes sont déphasés les uns avec les autres entre 0° et 180°.
